# EUROPÄISCHE PATENTSCHRIFT

(11) **EP 2 486 145 B1**
(45) Veröffentlichungstag und Bekanntmachung des Hinweises auf die Patenterteilung: **30.07.2014**
(21) Anmeldenummer: 10728593.4
(22) Anmeldetag: 17.06.2010
(51) Int. Cl.: C12Q 1/56

(54) **Verfahren zur Bestimmung von Faktor XIII mit Hilfe von Referenzmaterial auf Plasmabasis**
Method of determining factor XIII with the aid of plasma-based reference material.
Procédé de détermination du facteur XIII au moyen de matériel de référence à base de plasma.

(30) Priorität: 05.10.2009 DE 102009048199
(43) Veröffentlichungstag der Anmeldung: 15.08.2012
(73) Patentinhaber: Siemens Healthcare Diagnostics Products GmbH, 35041 Marburg (DE)
(72) Erfinder: CHRIST, Gerlinde, 35043 Marburg (DE); KAPPEL, Andreas, 61462 Koenigstein (DE); PECHMANN, Lena, 35102 Lohra (DE); VITZTHUM, Frank, 35094 Lahntal (DE); ZANDER, Norbert, 35039 Marburg (DE)
(86) Internationale Anmeldenummer: PCT/EP2010/003667
(87) Internationale Veröffentlichungsnummer: WO 2011/042072

(56) Entgegenhaltungen:
- EP-A2- 0 336 353
- MUSZBEK L ET AL: "KINETIC DETERMINATION OF BLOOD COAGULATION FACTOR XIII IN PLASMA" CLINICAL CHEMISTRY, AMERICAN ASSOCIATION FOR CLINICAL CHEMISTRY, WASHINGTON, DC, Bd. 31, Nr. 1, 1. Januar 1985 (1985-01-01) , Seiten 35-40, XP002036558 ISSN: 0009-9147
- FICKENSCHER K ET AL: "A PHOTOMETRIC ASSAY FOR BLOOD COAGULATION FACTOR XIII" THROMBOSIS AND HAEMOSTASIS, SCHATTAUER GMBH, DE; US, Bd. 65, Nr. 5, 6. Mai 1991 (1991-05-06), Seiten 535-540, XP009048407 ISSN: 0340-6245
- KARPATI L ET AL: "A modified, optimized kinetic photometric assay for the determination of blood coagulation factor XIII activity in plasma" CLINICAL CHEMISTRY, AMERICAN ASSOCIATION FOR CLINICAL CHEMISTRY, WASHINGTON, DC, Bd. 46, Nr. 12, 1. Januar 2000 (2000-01-01), Seiten 1946-1955, XP002317468 ISSN: 0009-9147

## Beschreibung

Die vorliegende Erfindung liegt auf dem Gebiet der in vitro-Diagnostik und betrifft ein Verfahren zur Bestimmung des Blutgerinnungsfaktors XIII (Faktor XIII, F XIII) mit Hilfe von Referenzmaterial auf Plasmabasis.

Faktor XIII ist ein Blutgerinnungsfaktor, der am Ende der Blutgerinnungskaskade wirkt und eine wichtige Rolle für den dauerhaften Wundverschluss spielt. In der letzten Phase der Blutgerinnung, der Fibrinbildung, spaltet Thrombin Fibrinogen. Die so entstehenden Fibrinmonomere aggregieren spontan zu langen Fasern und schließlich zu einem dichten, verzweigten Netz aus löslichen Fibrinpolymeren. Auch Faktor XIII wird durch Thrombin aktiviert, wodurch Faktor XIIIa entsteht. Faktor XIIIa bewirkt eine Quervernetzung der Fibrinpolymere, wodurch das Fibringerinnsel mechanisch stabiler, weniger deformierbar und resistenter gegen Auflösung durch Plasmin wird. Ein kongenitaler oder erworbener Faktor XIII-Mangel kann zu Blutungsneigung, Wundheilungsstörungen sowie zu Aborten führen. Aufgrund der klinischen Relevanz ist die Bestimmung von Faktor XIII zum Ausschluss oder zur Feststellung eines Faktor XIII-Mangels ein wichtiger Bestandteil der Gerinnungsdiagnostik.

Faktor XIIIa, die aktivierte Form des katalytisch inaktiven Proenzyms Faktor XIII, ist eine Transglutaminase, die die dreidimensionale Quervernetzung der Fibrinpolymere durch Ausbildung intermolekularer Amidbindungen zwischen Lysyl- und Glutaminyl-Aminosäureseitenketten der Fibrinmoleküle katalysiert. Bei dieser Reaktion entsteht Ammoniak (NH₃) bzw. Ammoniumionen (NH₄⁺) werden freigesetzt. Im Folgenden wird sowohl für Ammoniak als auch Ammoniumionen der Einfachheit halber der Begriff Ammoniak verwendet. Das Phänomen der Ammoniakentstehung wird in verschiedenen Testverfahren zur Bestimmung von Faktor XIII ausgenutzt: Muszbek, L. et al. [Clin. Chem. (1985) 31(1), 35-40] beschreiben ein Verfahren zur Bestimmung von Faktor XIII in defibrinierten Plasmaproben, wobei der Faktor XIII der Probe mit Thrombin zu Faktor XIIIa aktiviert wird. Ferner wird die Probe mit β-Casein und Ethylamin vermischt, die als Substrate für die Ausbildung intermolekularer Amidbindungen durch Faktor Xllla dienen. Um den bei dieser Reaktion freigesetzten Ammoniak quantitativ nachzuweisen, wird die Probe zusätzlich mit NADPH (Nicotinsäureamid-Adenin-Dinukleotid-Phosphat-Hydrid) und mit Komponenten einer NADPH-abhängigen Indikatorreaktion vermischt, nämlich mit Glutamatdehydrogenase (GLDH) und α-Ketoglutarat. In Anwesenheit von Ammoniak wandelt GLDH α-Ketoglutarat in Glutamat um. Diese Reaktion verbraucht zusätzlich NADPH, und es entsteht NADP+ (Nicotinsäureamid-Adenin-Dinukleotid-Phosphat), die oxidierte Form von NADPH. NADP+ hat ein anderes Absorptionsspektrum als NADPH, so dass sich die Absorption (Extinktion oder optische Dichte) des Testansatzes proportional zum NADPH-Verbrauch und damit proportional zur Ammmoniakmenge und damit proportional zur Faktor XIII-Menge bzw. -Aktivität ändert. Alternativ kann in diesem Testansatz NADH anstatt NADPH verwendet werden. Im Gegensatz zu NADP+ besitzt NADPH neben einem Absorptionsmaximum bei circa 260 nm ein Absorptionsmaximum bei circa 340 nm. Die exakte Lage von Absorptionsmaxima hängt in der Regel von verschiedenen Parametern ab, insbesondere von der Dielektrizitätskonstanten und dem pH-Wert der Lösung. Im Allgemeinen liegt das Absorptionsmaximum des NADPH im Bereich von 335 bis 345 nm. Die Messung der Änderung der Absorption des Testansatzes daher üblicherweise bei einer Wellenlänge von circa 340 ± 5 nm ermöglicht die quantitative Bestimmung des Faktor XIII in einer Probe.

EP 336 353 A2 bzw. Fickenscher et al. (Thromb Haemost. 1991, 65(5): 535-40) beschreiben ein ähnliches Verfahren, mit dem Faktor XIII über das freigesetzte Ammoniak quantifiziert wird. EP 336 353 A2 beschreibt ein Verfahren zur Bestimmung von Faktor XIII in unvorbehandelten, fibrinhaltigen Plasmaproben. Um die Entstehung störender Fibringerinnsel im Reaktionsansatz zu unterbinden, wird die Probe zusätzlich mit einem Fibrinaggregationshemmer vermischt. Der Faktor XIII der Probe wird mit Thrombin in Gegenwart von Ca²⁺-Ionen zu Faktor XIIIa aktiviert. Ferner wird die Probe mit einem synthetischen, glutaminhaltigen Peptid und Glycinethylester vermischt, die als Substrate für die Ausbildung intermolekularer Amidbindungen durch Faktor XIIIa dienen. Um den bei dieser Reaktion freigesetzten Ammoniak quantitativ nachzuweisen, wird die Probe zusätzlich mit NADH (Nicotinsäureamid-Adenin-Dinukleotid-Hydrid) und mit Komponenten einer NADH-abhängigen Indikatorreaktion vermischt, nämlich mit Glutamatdehydrogenase (GLDH) und α-Ketoglutarat. In Anwesenheit von Ammoniak wandelt GLDH α-Ketoglutarat in Glutamat um. Diese Reaktion verbraucht zusätzlich NADH, und es entsteht NAD+, die oxidierte Form von NADH. NAD+ hat ein anderes Absorptionsspektrum als NADH, so dass sich die Absorption des Testansatzes proportional zum NADH-Verbrauch und damit proportional zur Ammmoniakmenge und damit proportional zur enzmyatischen Aktivität des Faktor XIII und damit proportional zur Faktor XIII-Menge ändert. Alternativ kann in diesem Testansatz NADPH anstatt NADH verwendet werden. Die Messung der Änderung der Absorption des Testansatzes bei einer Wellenlänge von 340 nm ermöglicht die quantitative Bestimmung des Faktors XIII in einer Probe. Ein kommerzieller Test, der auf dem in EP 336 353 A2 beschriebenen Testprinzip beruht, ist der Berichrom® F XIII Test von Siemens Healthcare Diagnostics.

Karpati et al., 2000, offenbart ein Verfahren zur quantitativen Bestimmung von Faktor XIII in einer Plasmaprobe, wobei die Transglutaminaseaktivität des aktivierten Faktors XIIIa anhand des oxidativen Verbrauchs von NADPH im Testansatz gemessen wird. Karpati et al. offenbart nicht, dass NADH oder ein NAD(P)H-Analogon verwendet wird.

Zur Vereinfachung wird der Terminus NAD(P)H verwendet, wenn Ausführungen sowohl die phosphorylierte als auch die nicht-phosphorylierte Form von NADH betreffen, also wenn NADH und NADPH gleichermaßen gemeint sind. Der Terminus NAD(P)+ wird verwendet, wenn Ausführungen sowohl die phosphorylierte als auch die nicht-phosphorylierte Form von NADH im oxidierten Zustand betreffen, also wenn NAD+ und NADP+ gleichermaßen gemeint sind.

An Stelle von NADH oder NADPH können in den beschriebenen Testverfahren auch Analoga von NADH oder NADPH eingesetzt werden, sogenannte NAD(P)H-Analoga. Bevorzugt sind solche Analoga, die ein Absorptionsmaximum aufweisen, das oberhalb von 350 nm liegt. Ein Analogon ist eine Substanz, die die biologische Wirkung der physiologischen nachahmt, d.h. in diesem Fall beispielsweise eine Substanz, die wie NAD(P)+ bzw. NAD(P)H als Kosubstrat fungieren kann. Dabei muss es sich um ein Analogon handeln, bei dem die oxidierte und reduzierte Form unterschiedliche Absoprtionsmaxima haben, wobei das Absorptionsmaximum des NAD(P)H-Analogons oberhalb von 350 nm liegt. Vorzugsweise sind Strukturanaloga zu verwenden, bei denen die Nikotinamidgruppe gegen eine andere Gruppe ausgetauscht wurde. Bevorzugt sind hier zyklische Verbindungen, insbesondere heterozyklische Verbindungen, in ganz besonderer Weise bevorzugt sind Pyridinanaloga, d.h. beispielsweise, 3-Acetylpyridin, 3-(Carb)-Aldehydpyridine, Thionikotinamid, Selenonikotinamid, etc.

Die zuvor beschriebenen Verfahren aus dem Stand der Technik sowie andere Verfahren zur Bestimmung der Faktor XIII-Aktivität, die auf dem Nachweis der Ammoniakbildung im Testansatz beruhen, haben den Nachteil, dass in Proben mit niedrigen Faktor XIII-Konzentrationen häufig eine zu hohe Faktor XIII-Aktivität gemessen wird. Es wurde sogar beobachtet, dass in Plasmaproben von Patienten mit einem schweren erworbenen Faktor XIII-Mangel, in denen mittels immunologischer und anderer Verfahren kein Faktor XIII nachgewiesen werden konnte, mit dem Berichrom® F XIII Test eine Faktor XIII-Aktivität von 8-14 % nachgewiesen wurde (Lim, W. et al., J Thromb Haemost 2004; 2: 1017-1019). Eine korrekte Bestimmung der Faktor XIII-Aktivität ist jedoch gerade in Proben mit niedrigen Faktor XIII-Konzentrationen eine wesentliche Voraussetzung für eine bestmögliche Therapierung der Patienten mit Faktor XIII-Konzentrat.

Die Ursache für die falsch zu hoch bestimmten Faktor XIII-Aktivitäten ist unbekannt. Als mögliche Ursache wird ein Faktor Xllla-unabhängiger NADH- oder NADPH-Verbrauch diskutiert, möglicherweise hervorgerufen durch Enzyme, die in der Patientenprobe enthalten sind und die NADH bzw. NADPH als Kofaktor benutzen, oder durch unspezifische Ammoniakentstehung in der Patientenprobe, welche auch zu einem Faktor XIIIa-unabhängigen NADH- oder NADPH-Verbrauch führen würde (Ajzner, E and Muszbek, L.; J Thromb Haemost 2004; 2: 2075-2077). Zur Lösung des Problems der falsch zu hoch bestimmten Faktor XIII-Aktivitäten schlagen Ajzner & Muszbek vor, in einem Parallelansatz ein zweites Aliquot der Patientenprobe mit lodoacetamid zu vermischen und die Faktor XIII-Aktivität zu bestimmen. Iodoacetamid inhibiert die Transglutaminaseaktivität des aktivierten Faktors XIII. Die Extinktionsänderung, die in diesem Parallelansatz gemessen wird, entspricht dem Faktor XIIIa-unabhängigen NADH- oder NADPH-Verbrauch, der dann von der Aktivität, die in dem regulären, nicht mit lodoacetamid-behandelten Testansatz gemessen wird, abgezogen werden kann. Auf diese Weise kann die im regulären Testansatz gemessene Faktor XIII-Aktivität korrigiert werden.

Nachteilig bei diesem Verfahren zur Korrektur der Faktor XIII-Aktivität ist jedoch, dass jede Patientenprobe doppelt gemessen werden muss, einmal ohne und einmal mit lodoacetamid. Ein solches Vorgehen würde nicht nur das Testaufkommen, sondern auch die Kosten für eine Faktor XIII-Bestimmung verdoppeln. Zudem ist nicht geklärt, ob die Verwendung von lodoacetamid für den Routineeinsatz auf Gerinnungsanalyzern geeignet ist.

Der vorliegenden Erfindung lag damit die Aufgabe zugrunde, Mittel und Verfahren zur Bestimmung von Faktor XIII bereit zu stellen, die es ermöglichen, ohne die Messung eines Parallelansatzes Faktor XIII insbesondere in Plasmaproben mit niedriger Faktor XIII-Konzentration oder -Aktivität korrekt zu bestimmen.

Die Aufgabe wird dadurch gelöst, dass als Referenzmaterial für Faktor XIII-Aktivitäten von weniger als 100 % der Norm ausschließlich Plasma verwendet wird.

Bei den bekannten Verfahren zur Bestimmung von Faktor XIII in Plasmaproben wird üblicherweise ein Normalplasmapool, z. B. aus dem Plasma von in der Regel mindestens 20 offensichtlich gesunden Spendern, als Referenzmaterial verwendet. Die Faktor XIII-Aktivität dieses Normalplasmas wird als 100 % bzw. als Norm definiert oder sie wird mit der der Faktor XIII-Aktivität eines internationalen Referenzplasmas verglichen, wodurch dem Normalplasma ein Faktor XIII Aktivitätswert zugeordnet werden kann. Zur Bereitstellung von Referenzen mit geringerer Faktor XIII-Aktivität (< 100 %) werden Verdünnungen dieses Normalplasmas hergestellt. Als Verdünnungsmedium werden üblicherweise wässrige Lösungen, wie z. B. 0,9 % NaCl-Lösung verwendet. Zum Beispiel wird ein Teil Normalplasma mit zwei Teilen eines geeigneten Puffers vermischt (1:2 Verdünnung) und als Referenz mit einer Faktor XIII-Aktivität von 33,33 % der Norm definiert. Durch Messung des Normalplasmas und einer Reihe von Verdünnungen des Normalplasmas mit Hilfe des zu standardisierenden Faktor XIII-Tests wird eine Referenzkurve (Kalibrationskurve) erstellt, indem die definierten Faktor XIII-Aktivitäten (z. B. in % der Norm) den gemessenen Rohwerten zugeordnet werden. Für Patientenproben kann dann in dem standardisierten Testsystem der Rohwert gemessen werden und schließlich anhand der Referenzkurve in einen kalibrierten Wert bzw. ein standardisiertes Testergebnis, z. B. in % der Norm umgerechnet werden.

Es wurde festgestellt, dass durch die Verwendung von Referenzen für den Normwert der Faktor XIII-Aktivität (100 %) und für Faktor XIII-Aktivitäten unterhalb des Normwerts (< 100 %), die ausschließlich aus Plasma bestehen, eine Referenzkurve erhalten wird, die eine korrekte Bestimmung von Faktor XIII, insbesondere in Plasmaproben mit niedriger Faktor XIII-Konzentration oder -Aktivität, mittels eines Verfahrens, bei dem die Transglutaminaseaktivität des aktivierten Faktors XIIIa anhand des NAD(P)H-Verbrauchs im Testansatz gemessen wird, ermöglicht.

Ein Gegenstand der vorliegenden Erfindung ist ein Verfahren zur quantitativen Bestimmung von Faktor XIII in einer Plasmaprobe, wobei die Transglutaminaseaktivität des aktivierten Faktors Xllla anhand des oxidativen NAD(P)H-Verbrauchs im Testansatz gemessen wird und wobei der ermittelte Messwert mit Referenzwerten verglichen wird, welche mit Hilfe von Referenzmaterialien bestimmt sind, wobei zumindest die Referenzmaterialien, welche eine gegenüber der Norm verminderte Faktor XIII-Aktivität aufweisen, aus Plasma bestehen.

Unter einem Verfahren zur quantitativen Bestimmung von Faktor XIII in einer Plasmaprobe, bei dem die Transglutaminaseaktivität des aktivierten Faktors XIIIa anhand des oxidativen NAD(P)H-Verbrauchs im Testansatz gemessen wird, ist beispielsweise ein Verfahren zu verstehen, bei dem eine Plasmaprobe
I. mit einer Substanz oder einem Substanzgemisch zur Aktivierung des Faktor XIII zu Faktor XIIIa,
II. mit einem Akzeptorsubstrat für Faktor Xllla mit mindestens einer Glutaminylgruppe,
III. mit einem Aminogruppendonorsubstrat für Faktor XIIIa,
IV. mit NAD(P)H oder einem NAD(P)H-Analogon und
V. mit einem Mittel, das in Gegenwart von Ammoniak NAD(P)H zu NADP+ zu oxidieren vermag,
vermischt wird und die Änderung der Absorption des Testansatzes gemessen wird.

Als Substanz zur Aktivierung des Faktor XIII zu Faktor Xllla eignet sich insbesondere Thrombin, beispielsweise humanen oder bovinen Ursprungs oder auch rekombinant hergestelltes Thrombin, bevorzugterweise in Gegenwart von Calciumionen. Ebenfalls geeignet sind Substanzen oder Substanzgemische wie beispielsweise Faktor Xa, das Schlangengift Ecarin oder eine Mischung aus Tissue factor, Phospholipiden und Ca²⁺-Ionen, die indirekt eine Aktivierung des Faktors XIII bewirken, indem sie das in der Probe enthaltene Prothrombin direkt oder indirekt zu Thrombin aktivieren, welches dann wiederum den Faktor XIII aktiviert.

Unter dem Begriff "Akzeptorsubstrat für Faktor XIIIa mit mindestens einer Glutaminylgruppe" ist ein Polypeptid oder Peptidmimetikum zu verstehen, das mindestens eine Glutaminylgruppe, beispielsweise von der Aminosäure Glutaminsäureamid aufweist. Bekannte Akzeptorsubstrate für Faktor XIIIa sind z. B. β-Casein sowie eine Vielzahl synthetischer Peptide. Geeignete synthetische Peptide sind beispielweise in EP 314 023 A2 beschrieben.

Unter dem Begriff "Aminogruppendonorsubstrat für Faktor XIIIa" sind insbesondere primäre Amine zu verstehen. Bevorzugte primäre Amine sind Ethanolamin, Putrescin, Cadaverin, Diaminoethan, Aminoethan. Besonders bevorzugte primäre Amine sind Glycinethylester oder Glycinmethylester.

Der Begriff "NADH" ist die Abkürzung für die Verbindung Nicotinsäureamid-Adenin-Dinukleotid-Hydrid. Unter einem NADH-Verbrauch ist im Sinne der vorliegenden Erfindung die Oxidation von NADH zu NAD+ (Nicotinsäureamid-Adenin-Dinukleotid) zu verstehen. Der Begriff "NADH" ist breit zu verstehen und umfasst auch die folgenden NADH-Analoga, die in analoger Weise wie NADH oxidierbar sind und die ebenfalls in oxidierter Form über andere optische Eigenschaften verfügen als ihre reduzierte Form, so dass ihr Verbrauch photometrisch gemessen werden kann: NADPH (Nicotinsäureamid-Adenin-Dinukleotid-Phosphat-Hydrid, oxidierbar zu NADP+), Thio-NADH (Thio-Nicotinsäureamid-Adenin-Dinukleotid-Hydrid, oxidierbar zu Thio-NAD+), Thio-NADPH (Thio-Nicotinsäureamid-Adenin-Dinukleotid-Phosphat-Hydrid, oxidierbar zu Thio-NADP+), Seleno-NADH (Seleno-Nicotinsäureamid-Adenin-Dinukleotid-Hydrid, oxidierbar zu Seleno-NAD+) und Seleno-NADPH (Seleno-Nicotinsäureamid-Adenin-Dinukleotid-Phosphat-Hydrid, oxidierbar zu Seleno-NADP+). Bei Verwendung von NADH oder NADPH wird die Änderung der Extinktion infolge der Oxidation zu NAD+ bzw. NADP+ bei einer Wellenlänge von etwa 340 nm gemessen. Bei Verwendung von Thio-NADH, Thio-NADPH, Seleno-NADH oder Seleno-NADPH wird die Änderung der Extinktion infolge der Oxidation zu Thio-NAD+, Thio-NADP+, Seleno-NAD+ oder Seleno-NADP+ bei Wellenlängen von etwa 340 nm bis etwa 430 nm gemessen.

Ein "Mittel, das in Gegenwart von Ammoniak NADH zu NAD+ zu oxidieren vermag" ist bevorzugterweise ein Enzym/Substratsystem, welches ein Enzym und ein Substrat für das Enzym umfasst, wobei das Enzym katalytisch auf das Substrat einwirkt und dabei in Gegenwart von Ammoniak NADH zu NAD+ bzw. die obengenannten NADH-Analoga oxidiert. Geeignete Enzym/Substratsysteme sind z. B: das Glutamatdehydrogenase/ Ketoglutarat-System oder das Alanindehydrogenase/Pyruvat-System oder das Serin-2-dehydrogenase/3-Hydroxypyrovat-System oder das Valindehydrogenase/3-Methyl-2-Oxobutanoat-System oder das Leucindehydrogenase/4-Methyl-2-Oxopentanoat-System oder das Glycindehydrogenase/Glyoxylat-System oder das Lysindehydrogenase/1,2-Didehydropiperidin-2-Carboxylat-System oder das Phenylalanin/Phenylpyruvat-System oder das Aspartatdehydrogenase/Oxaloacetat-System oder das Glucose-6-Phosphat-Dehydrogenase/D-Glucono-1,5-lakton-6-Phosphat-System.

Bevorzugt wird die Plasmaprobe zusätzlich mit einem Fibrinaggregationshemmer vermischt. Fibrinaggregationshemmer sind Substanzen, die die Aggregation von Thrombininduzierten Fibrinmonomeren verhindern. Auf diese Weise wird die Entstehung eines Fibringerinnsels in einer fibrinogenhaltigen Probe verhindert, welches ansonsten die Absorptionsmessung des Testansatzes negativ beeinträchtigen würde. Bevorzugte Fibrinaggregationshemmer sind synthetische Peptide, wie z. B. ein Peptid der Sequenz Gly-Pro-Arg-Pro (kommerziell erhältlich als Pefabloc®FG, Pentapharm, Schweiz). Weitere bevorzugte Peptide, die als Fibrinaggregationshemmer verwendet werden können, insbesondere das bevorzugte Peptid der Sequenz Gly-Pro-Arg-Pro-Ala sind in EP 456 152 A2 beschrieben.

Bevorzugt wird die Probe zusätzlich mit einer Heparinneutralisierenden Substanz vermischt, beispielsweise mit Hexadimethrinbromid (auch bekannt als Polybrene®), um die Thrombin-inhibierende Wirkung von Heparin, das z. B. in Proben von Heparin-therapierten Patienten vorhanden sein kann, auszuschalten.

Die Komponenten I bis V, die mit der Probezu einem Testansatz vermischt werden, können jeweils einzeln, d. h. in Form einzelner Reagenzien und nacheinander mit der Probe vermischt werden; sie können aber auch in einem einzigen Reagenz vereinigt sein, welches in einem einzigen Pipettierschritt mit der Probe vermischt wird. Das Reagenz oder die Reagenzien umfassen vorzugsweise eine Puffermatrix, in der die Substanzen gelöst sind. Eine geeignete Puffermatrix enthält z. B. HEPES, Bicin, NaCl, Albumin und/oder Konservierungsmittel, wie z. B. Natriumazid und hat vorzugsweise einen pH-Wert von 6.0 bis 9.0, besonders bevorzugt von 6.5 bis 8.5. Da Calciumionen für die Aktivierung von F XIII notwendig sind, enthält die Puffermatrix ferner ein Calciumsalz, bevorzugterweise Calciumchlorid. Das Vermischen des Reagenzes oder der Reagenzien mit der Probe kann manuell oder an automatischen Gerinnungsmessgeräten durchgeführt werden.

Sofern das Glutamatdehydrogenase/Ketoglutarat-System als Mittel, das in Gegenwart von Ammoniak NADH zu NAD zu oxidieren vermag, in dem erfindungsgemäßen Verfahren eingesetzt wird, ist die Menge an Glutamatdehydrogenase, die dem Testansatz zugegeben wird, so gewählt, dass die Endkonzentration im Testansatz 10 - 500 IU/mL, bevorzugterweise 40 - 240 IU/mL beträgt.

Als Probenmaterial eignet sich insbesondere Fibrinogen-haltiges Plasma. Aber auch in defibriniertem Plasma kann Faktor XIII gemäß dem erfindungsgemäßen Verfahren bestimmt werden.

Die Messung der Absorptionsänderung (ΔA) des Testansatzes erfolgt mit Hilfe eines Photometers umfassend eine Lichtquelle, die einen Lichtstrahl durch den zu messenden Testansatz sendet und einen Detektor, der die Intensität des durchgetretenen Lichts misst und in ein elektrisches Signal umwandelt. Die Messung der Absorptionsänderung erfolgt je nachdem ob NADH bzw. NADPH oder Thio-NADH, Thio-NADPH, Seleno-NADH bzw. Seleno-NADPH verwendet wird, mit Licht einer Wellenlänge von etwa 340 nm bis etwa 430 nm. Die sich pro Zeiteinheit ändernde Absorption korreliert mit der Faktor XIII-Aktivität. Die Abnahme der Absorption (A) des Testansatzes durch den NADH- bzw. NADH-Analogon-Verbrauch ist insbesondere im linearen Bereich der Reaktionskinetik direkt proportional zur Faktor XIII-Aktivität.

Der ermittelte Messwert einer Plasmaprobe ist zunächst ein Rohwert, welcher anschließend mit Referenzwerten verglichen wird, die mit Hilfe von Referenzmaterialien bestimmt wurden. Unter einem Referenzmaterial ist im Sinne der vorliegenden Erfindung ein Material zu verstehen, dessen Faktor XIII-Konzentration bzw. -Aktivität bekannt ist.

Gemäß der vorliegenden Erfindung bestehen zumindest die Referenzmaterialien, welche eine gegenüber der Norm verminderte Faktor XIII-Aktivität aufweisen (< 100 % d. N.), aus Plasma. Unter dem Begriff "Plasma", ist der flüssige, zellfreie Teil von Blut eines oder mehrerer Spender, bevorzugterweise menschlicher Spender, zu verstehen, welcher -wie in der Gerinnungsanalytik üblich- durch Abtrennung der zellulären Blutbestandteile aus antikoaguliertem Vollblut gewonnen wurde. Um die Blutgerinnung in einer Vollblutprobe zu unterbinden, wird jede Blutprobe -häufig schon während der Blutentnahme- mit einem Antikoagulanz, vorzugsweise mit einer Natriumcitratlösung, vermischt. Andere gängige Antikoagulanzien sind Natriumheparin, Lithiumheparin, EDTA oder Citrat-Phosphat-Dextrose (CPD). Jedes Plasma umfasst damit unweigerlich einen gewissen Volumenanteil Antikoagulanz.

Als Normalplasma wird eine Mischung von Plasmen mehrerer offensichtlich gesunder Spender (Plasmapool) bezeichnet. Ein Normalplasma weist per definitionem eine Faktor XIII-Konzentration und damit eine Faktor XIII-Aktivität von 100 % auf. Ein Faktor XIII-defizientes Plasma ist ein Plasma, das keine oder zumindest nicht mehr als 1 % der normalen Faktor XIII-Aktivität aufweist. Faktor XIII-defizientes Plasma kann beispielsweise aus Patienten mit einem entsprechenden Phänotyp oder durch selektive Immunadsorption des Faktors XIII mittels spezifischer Antikörper aus einem Normalplasma gewonnen werden. Der Vollständigkeit halber wird darauf hingewiesen, dass die tatsächliche Faktor XIII-Aktivität verschiedener Normalplasmen je nach Hersteller, Charge, Abfüllung etc. variiert. Die tatsächliche Faktor XIII-Aktivität eines Normalplasmas wird durch den Vergleich mit einem internationalen Standardplasma für F XIII bestimmt. Die tatsächliche Faktor XIII-Aktivität eines Normalplasmas liegt in der Regel zwischen 80 % und 120 % des internationalen Standardplasmas.

Ein erfindungsgemäßes Referenzmaterial, welches eine gegenüber der Norm verminderte Faktor XIII-Aktivität aufweist, kann aus einer Mischung von Normalplasma und Faktor XIII-defizientem Plasma bestehen. Dazu wird Normalplasma mit einem Faktor XIII-defizienten Plasma in solchen Teilen vermischt, dass die erhaltene Mischung die gewünschte Faktor XIII-Aktivität aufweist. Wird zum Beispiel ein Teil Normalplasma mit zwei Teilen eines Faktor XIII-defizienten Plasmas vermischt (1:2 Verdünnung), erhält man ein Referenzmaterial mit einer Faktor XIII-Aktivität von 33,33 % der Norm. Ein erfindungsgemäßes Referenzmaterial, welches eine gegenüber der Norm verminderte Faktor XIII-Aktivität aufweist, kann ferner aus einem Faktor XIII-defizienten Plasma bestehen, dem gereinigter, d. h. isolierter Faktor XIII in solcher Menge zugesetzt wurde, dass eine gewünschte Faktor XIII-Aktivität erzielt wird. Geeignet ist humaner, tierischer oder rekombinant hergestellter Faktor XIII. Ein erfindungsgemäßes Referenzmaterial, welches keine Faktor XIII-Aktivität aufweist (≤ 1%) besteht bevorzugterweise aus Faktor XIII-defizientem Plasma.

Erfindungsgemäß werden für die Ermittlung der Referenzwerte bzw. für die Erstellung der Referenzkurve für Faktor XIII-Aktivitäten unterhalb des Normwerts (< 100 %) ausschließlich Referenzmaterialien verwendet, die aus Plasma bestehen. In einer bevorzugten Ausführungsform wird für die Ermittlung des Referenzwertes für eine Faktor XIII-Aktivität entsprechend dem Normwert (100 %) ein Referenzmaterial verwendet, das aus Normalplasma besteht. In einer ebenfalls bevorzugten Ausführungsform werden für die Ermittlung der Referenzwerte bzw. für die Erstellung der Referenzkurve für Faktor XIII-Aktivitäten oberhalb des Normwerts (> 100 %) Referenzmaterialien verwendet, die aus Normalplasma bestehen, welchem isolierter Faktor XIII in solcher Menge zugesetzt ist, dass eine gewünschte Faktor XIII-Aktivität oberhalb des Normwerts erzielt wird.

Ein weiterer Gegenstand der vorliegenden Erfindung ist die Verwendung von Faktor XIII-defizientem Plasma zur Herstellung von Referenzmaterial, das für die Durchführung eines Verfahrens zur quantitativen Bestimmung von Faktor XIII geeignet ist. Wie bereits beschrieben, kann das Faktor XIII-defiziente Plasma als Verdünnungsmedium für Normalplasma verwendet werden, um so Referenzmaterialien herzustellen, die aus Plasma bestehen und eine Faktor XIII-Aktivität unterhalb des Normwerts (< 100 %) aufweisen. Das Faktor XIII-defiziente Plasma kann jedoch auch als Matrix verwendet werden, der gereinigter, d. h. isolierter Faktor XIII in solcher Menge zugesetzt wird, dass ein Referenzmaterial ensteht, das eine gewünschte Faktor XIII-Aktivität unterhalb oder oberhalb des Normwerts aufweist.

Ein weiterer Gegenstand der vorliegenden Erfindung ist ein Referenzmaterial mit einer gegenüber der Norm verminderten Faktor XIII-Aktivität (< 100 %), das aus einer Mischung von Normalplasma und Faktor XIII-defizientem Plasma besteht oder das aus einem Faktor XIII-defizientem Plasma besteht, welchem gereinigter, d. h. isolierter Faktor XIII in solcher Menge zugesetzt ist, dass es eine Faktor XIII-Aktivität unterhalb des Normwerts aufweist. Ein erfindungsgemäßes Referenzmaterial kann in flüssiger oder in lyophilisierter Form bereitgestellt werden.

Hierin erwähnt ist ein Kit, welches ein Normalplasma als separate Einheit und ein Faktor XIII-defizientes Plasma als separate Einheit jeweils in flüssiger oder lyophilisierter Form enthält. Mit Hilfe des Normalplasmas kann der Referenzwert für den Normwert der Faktor XIII-Aktivität (100 %) ermittelt werden. Mit Hilfe des Faktor XIII-defizienten Plasmas kann der Referenzwert für eine fehlende Faktor XIII-Aktivität (0 %) ermittelt werden. Mit Hilfe von Mischungen der beiden Plasmen, die vom Anwender nach Wunsch hergestellt werden können, können Referenzwerte für jede gegenüber der Norm verminderten Faktor XIII-Aktivität (< 100 %) ermittelt werden.

Weiterhin erwähnt ist ein Kit, welches mindestens ein Referenzmaterial als separate Einheit in flüssiger oder lyophilisierter Form enthält, welches eine gegenüber der Norm verminderte Faktor XIII-Aktivität (< 100 %) aufweist und aus Plasma besteht, bevorzugt aus einer Mischung von Normalplasma und Faktor XIII-defizientem Plasma. Bevorzugterweise enthält ein erfindungemäßes Testkit ferner ein Normalplasma als separate Einheit und/oder ein Faktor XIII-defizientes Plasma als separate Einheit.

### Figurenbeschreibung

Figur 1
   Kalibrationskurven des NADH-basierten Berichrom® Faktor XIII-Testes bzw. des Thio-NADH-basierten Faktor XIII-Testes gemäß dem Stand der Technik mit NaCl-Lösung als Verdünnungsmedium für das Standardplasma.
Figur 2
   Kalibrationskurven des NADH-basierten Berichrom® Faktor XIII-Testes bzw. des Thio-NADH-basierten Faktor XIII-Testes erfindungsgemäß mit Faktor XIIIdefizientem Plasma als Verdünnungsmedium für das Standardplasma.
Figur 3
   Vergleich der Wiederfindung von Proben unterschiedlicher Faktor XIII-Aktivität mit Hilfe des NADH-basierten Berichrom® Faktor XIII-Testes, durchgeführt mit NaCl oder Faktor XIII-defizientem Plasma als Verdünnungsmedium für das Standardplasma.
Figur 4
   Vergleich der Wiederfindung von Proben unterschiedlicher Faktor XIII-Aktivität mit Hilfe des Thio-NADH-basierten Faktor XIII-Testes, durchgeführt mit NaCl oder Faktor XIII-defizientem als Verdünnungsmedium für das Standardplasma.

### Beispiele

### Beispiel 1:

### Bestimmung von Faktor XIII

In dem folgenden Beispiel wurde der Faktor XIII-Gehalt von Plasmaproben mit Reagenzien bestimmt, die entweder NADH oder Thio-NADH als aktive Komponente enthalten. Das auf NADH basierende Reagenz ist in EP 336 353 A2 bzw. Fickenscher et al. (Thromb Haemost. 1991, 65(5): 535-40) in seiner Zusammensetzung beschrieben und wird von der Firma Siemens Healthcare Diagnostics (Deutschland) als Berichrom® Faktor XIII Reagenz vertrieben. Das auf Thio-NADH basierende Reagenz hat folgende Zusammensetzung:

### Aktivatorreagenz (pH 8.3):

- 292 µM Thio-NADH (Oriental Yeast Company, Rotterdam, Niederlande)
- Thrombin vom Rind (10 IU/mL)
- Gly-Pro-Arg-Pro-Ala-amid als Fibrinaggregationsinhibitor (2g/L)
- Calciumchlorid (1,2 g/L)
- Hexadimethrinbromid (10 mg/L)
- Albumin vom Rind
- Bicin-Puffer (100 mmol/L)

### Nachweisreagenz (pH 6.5):

- Glutamatdehydrogenase 160 U/mL)
- Leu-Gly-Pro-Gly-Gln-Ser-Lys-Val-Ile-Gly-amid als F XIII Akzeptorsubstrat (2,4 g/L)
- ADP
- Glycinethylester (1,4 g/L)
- α-Ketoglutarat (2,7 g/L)
- Albumin vom Rind
- HEPES-Puffer (10 mmol/L)

Für die Teste wurden auf dem BCS® XP Gerinnungsanalyzer (Siemens Healthcare Diagnostics Products GmbH, Marburg, Deutschland) von den jeweiligen Reagenzien 75 µL Aktivator-Reagenz, 75 µL Nachweis-Reagenz und 15 µL einer Plasmaprobe in einer Küvette vereint und bei 37 °C inkubiert. Nach 5 Minuten wurde die Messung der Extinktion gestartet. Testansätze mit dem NADH-Reagenz des Berichrom® Faktor XIII Testes wurden mit Licht einer Wellenlänge von 340 nm gemessen. Testansätze mit dem Thio-NADH-Reagenz wurden mit Licht einer Wellenlänge von 405 nm gemessen. Zur Auswertung wurde die Änderung der Extinktion pro Minute in einem Zeitfenster von 60 Sekunden - 350 Sekunden nach dem Start der Messung berechnet. Zur Kalibration, d. h. zur Ermittlung von Referenzwerten wurde Normalplasma (Standard Humanplasma, Siemens Healthcare Diagnostics Products GmbH, Marburg, Deutschland) als Standard mit einer Faktor XIII-Konzentration von 100 % d. N. (Prozent der Norm) verwendet (verglichen mit dem internationalen Standard für F XIII enthielt dieses Normalplasma 95 % F XIII). Kalibrationspunkte mit niedrigerer Faktor XIII-Konzentration, d. h. Referenzwerte, welche eine gegenüber der Norm verminderte Faktor XIII-Aktivität repräsentieren, wurden erfindungsgemäß durch Verdünnung des Standards mit Faktor XIII-defizientem Plasma beziehungsweise gemäß dem Stand der Technik durch Verdünnung des Standards mit 0,9 % NaCl-Lösung erhalten. Faktor XIII-defizientes Plasma wurde von Innovative Research (USA) bezogen bzw. mit monoklonalen Antikörpern gegen die Faktor XIII alpha- bzw. beta-Ketten in Analogie zu dem in EP 0 826 965 A1 beschriebenen Verfahren hergestellt. Kalibrationspunkte mit höherer Faktor XIII-Konzentration als die des Standards, d. h. Referenzwerte, welche eine gegenüber der Norm erhöhte Faktor XIII-Aktivität repräsentieren, wurden durch ein erhöhtes Volumen des Standardplasmas im Testansatz erhalten.

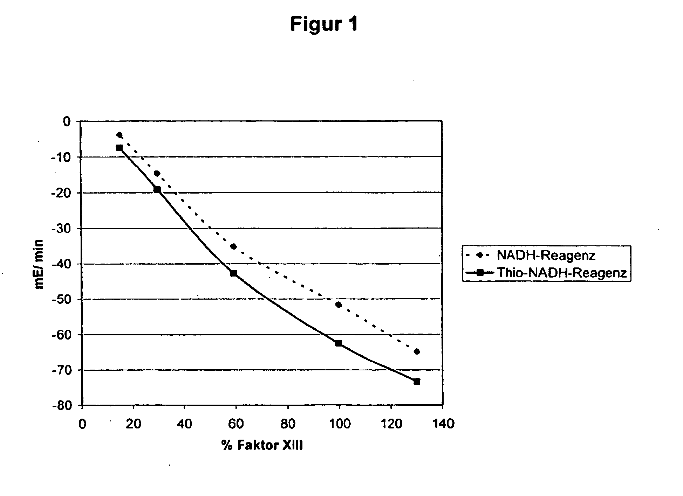

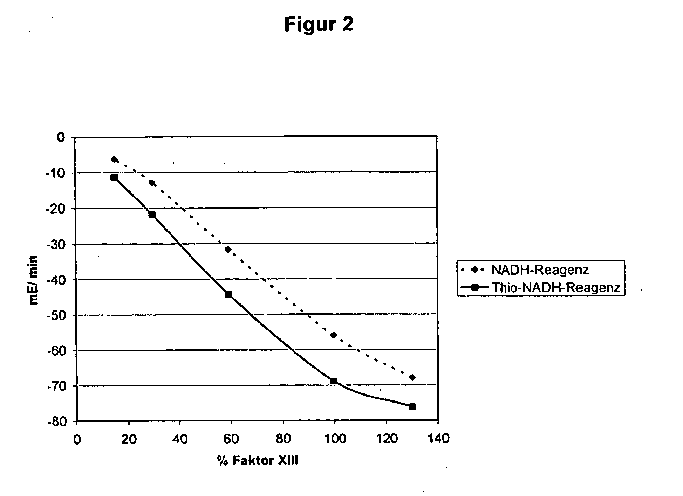

Typische Kalibrationskurven der mit den beiden Reagenzien durchgeführten Teste mit NaCl-Lösung als Verdünnungsmedium sind in Figur 1 gezeigt. Typische Kalibrationskurven der mit den beiden Reagenzien erfindungsgemäß durchgeführten Teste mit Faktor XIII-defizientem Plasma als Verdünnungsmedium sind in Figur 2 gezeigt.

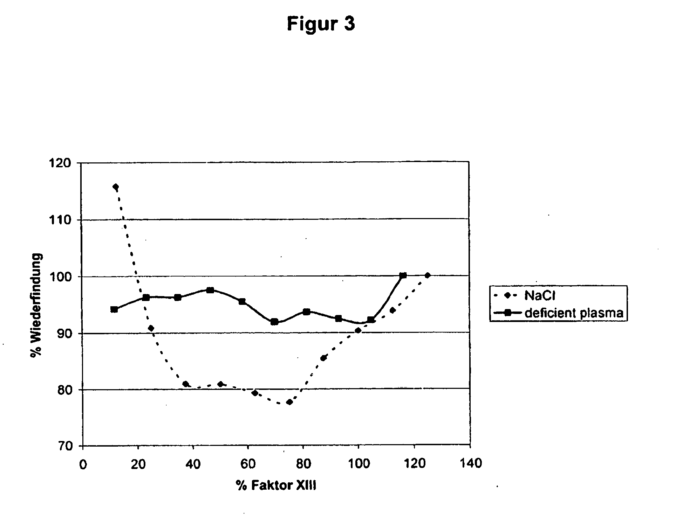

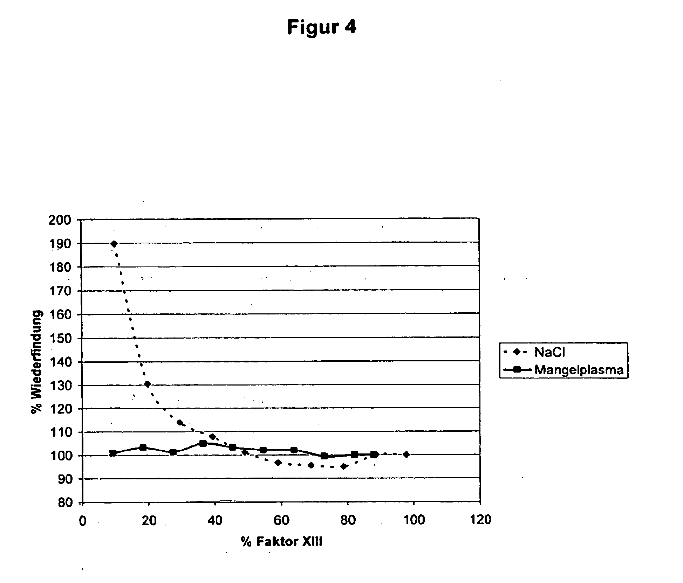

Anschließend wurden mit den beiden Reagenzien Proben mit unterschiedlichem Faktor XIII Gehalt gemessen, und die Testergebnisse wurden anhand der jeweiligen Kalibrationskurven ausgewertet. Die Proben wurden durch Mischen eines Plasmapools mit bekannter hoher Faktor XIII Aktivität mit einem Faktor XIII-defizienten Plasma in verschiedenen Verhältnissen hergestellt. Aus den Faktor XIII Aktivitätswerten der Ausgangsplasmen sowie dem Mischverhältnis wurde der theoretische Faktor XIII Aktivitätswert jeder Probe berechnet. Nach Messung der Proben wurde durch den Vergleich der tatsächlich ermittelten Messwerte mit den vorher theoretisch berechneten Faktor XIII Aktivitätswerten die prozentuale Wiederfindung jeder Probe berechnet. Eine Wiederfindung, die größer als 100 % ist, ist gleichbedeutende mit einem falsch zu hohen Testergebnis. Wie in Figur 3 gezeigt, war für den NADH-basierten Berichrom® Faktor XIII Test die Wiederfindung bei Proben mit einer Faktor XIII-Aktivität von weniger als 20 % näher an 100 %, wenn erfindungsgemäß Faktor XIII Mangelplasma an Stelle des für den Test vorgeschriebenen NaCl als Verdünnungsmedium für den Standard verwendet wurde. Wie in Figur 4 gezeigt, ist für den Thio-NADH basierten Test die Verbesserung der Wiederfindung von Proben mit niedrigem Faktor XIII Gehalt sogar noch ausgeprägter.

## Patentansprüche

1. Verfahren zur quantitativen Bestimmung von Faktor XIII in einer Plasmaprobe, wobei die Transglutaminaseaktivität des aktivierten Faktors XIIIa anhand des oxidativen Verbrauchs von NAD(P)H oder eines NAD(P)H-Analogons im Testansatz gemessen wird und der ermittelte Messwert mit Referenzwerten verglichen wird, welche mit Hilfe von Referenzmaterialien bestimmt sind, **dadurch gekennzeichnet, dass** zumindest die Referenzmaterialien, welche eine gegenüber der Norm verminderte Faktor XIII-Aktivität aufweisen, aus Plasma bestehen.

2. Verfahren gemäß Anspruch 1, wobei mindestens ein Referenzmaterial, welches eine gegenüber der Norm verminderte Faktor XIII-Aktivität aufweist, aus einer Mischung von Normalplasma und Faktor XIII-defizientem Plasma besteht.

3. Verfahren gemäß einem der vorhergehenden Ansprüche, wobei mindestens ein Referenzmaterial, welches eine gegenüber der Norm verminderte Faktor XIII-Aktivität aufweist, aus Faktor XIII-defizientem Plasma besteht, dem isolierter Faktor XIII zugesetzt ist.

4. Verfahren gemäß einem der vorhergehenden Ansprüche, wobei das Referenzmaterial, welches keine Faktor XIII-Aktivität aufweist, aus Faktor XIII-defizientem Plasma besteht.

5. Verfahren gemäß einem der vorhergehenden Ansprüche, wobei das Referenzmaterial, welches eine Faktor XIII-Aktivität von 100 % aufweist, aus Normalplasma besteht.

6. Verfahren gemäß einem der vorhergehenden Ansprüche, wobei mindestens ein Referenzmaterial, welches eine gegenüber der Norm erhöhte Faktor XIII-Aktivität aufweist, aus Normalplasma besteht, dem isolierter Faktor XIII zugesetzt ist.

7. Verfahren gemäß einem der vorhergehenden Ansprüche, wobei die Transglutaminaseaktivität des aktivierten Faktors XIIIa anhand des oxidativen Verbrauchs eines NAD(P)H-Analogons im Testansatz gemessen wird und wobei das NAD(P)H-Analogon Thio-NAD(P)H oder Seleno-NAD(P)H ist.

## Claims

1. Method for the quantitative determination of factor XIII in a plasma simple, where the transglutaminase activity of the activated factor XIIIa is measured via the oxidative consumption of NAD(P)H or an NAD(P)H analog in the test mixture and the measured value determined is compared to reference values which are determined with the aid of reference materials, **characterized in that** at least the reference materials having a reduced factor XIII activity compared to the norm consist of plasma.

2. Method according to Claim 1, where at least one reference material having a reduced factor XIII activity compared to the norm consists of a mixture of normal plasma and factor XIII-deficient plasma.

3. Method according to any of the preceding claims, where at least one reference material having a reduced factor XIII activity compared to the norm consists of factor XIII-deficient Plasma with added isolated factor XIII.

4. Method according to any of the preceding claims, where the reference material having no factor XIII activity consists of factor XIII-deficient plasma.

5. Method according to any of the preceding claims, where the reference material having a factor XIII activity of 100% consists of normal plasma.

6. Method according to any of the preceding claims, where at least one reference material having a factor XIII activity higher than the norm consists of normal plasma with added isolated factor XIII.

7. Method according to any of the preceding claims, where the transglutaminase activity of the activated factor XIIIa is measured via the oxidative consumption of an NAD(P)H analog in the test mixture and where the NAD(P)H analog is thio-NAD(P)H or seleno-NAD(P)H.

## Revendications

1. Procédé de détermination quantitative du facteur XIII dans un échantillon de plasma, en mesurant l'activité transglutaminase du facteur XIIIa activé, au moyen de la consommation par oxydation de NAD(P)H ou d'un analogue de NAD(P)H, dans une opération d'essai et en comparant la valeur de mesure déterminée à des valseurs témoins qui sont déterminées à l'aide de matières témoins, **caractérisé en ce qu'**au moins les matières témoins, qui ont une activité de facteur XIII diminuée par rapport à la norme, sont en plasma.

2. Procédé suivant la revendication 1, dans lequel au moins une matière témoin, qui a une activité de facteur XIII diminuée par rapport à la norme, est en un mélange de plasma normal et de plasma déficient en facteur XIII.

3. Procédé suivant l'une des revendications précédentes, dans lequel au moins une matière témoin, qui a une activité de facteur XIII diminués par rapport à la norme, est en un plasma déficient en facteur XIII, auquel du facteur XIII isolé est ajouté.

4. Procédé suivant l'une des revendications précédentes, dans lequel la matière témoin, qui n'a pas d'activité de facteur XIII, est en un plasma déficient en facteur XIII.

5. Procédé suivant l'une des revendications précédentes, dans lequel la matière témoin, qui a une activité de facteur XIII de 100%, est en plasma normal.

6. Procédé suivant l'une des revendications précédentes, dans lequel au moins une matière témoin, qui a une activité de facteur XIII plus élevée que la norme, est en Plasma normal, auquel du facteur XIII isolé est ajouté.

7. Procédé suivant l'une des revendications précédentes, dans lequel on mesure l'activité de transglutaminase du facteur XIIIa activé, au moyen de la consommation par oxydation d'un analogue de NAD(P)H dans une opération d'essai et dans lequel l'analogue de NAD(P)H est du thio-NAD(P)H ou du séléno-NAD(P)H.
